# EUROPÄISCHE PATENTSCHRIFT

(11) **EP 2 163 263 B1**
(45) Veröffentlichungstag und Bekanntmachung des Hinweises auf die Patenterteilung: **01.04.2020**
(21) Anmeldenummer: 09170279.5
(22) Anmeldetag: 15.09.2009
(51) Int. Cl.: A61L 2/28, G01N 21/64

(54) **Vorrichtung zum visuellen Erkennen der Verteilung von lumineszierenden Substanzen**
Device for visual recognition of the distribution of luminescent substances
Dispositif de reconnaissance visuelle de la répartition de substances luminescentes

(30) Priorität: 16.09.2008 DE 202008012267 U
(43) Veröffentlichungstag der Anmeldung: 17.03.2010
(73) Patentinhaber: KBD Gesellschaft für Unternehmensberatung, Marketing und Vertrieb mbH, 69469 Weinheim (DE)
(72) Erfinder: Bartling-Dudziak, Karin, 69469 Weinheim (DE)
(74) Vertreter: Hoffmann Eitle

(56) Entgegenhaltungen:
- EP-A- 0 913 681
- CH-A5- 693 765
- DE-A1- 10 247 605
- DE-U1-202006 011 931
- GB-A- 402 136
- US-A1- 2005 243 538
- US-B1- 7 396 148

## Beschreibung

### Technisches Gebiet

Die Erfindung betrifft eine Vorrichtung zum visuellen Erkennen und/oder Darstellen der Verteilung von lumineszierenden, insbesondere fluoreszierenden Substanzen, umfassend ein seitlich öffnungsfähiges Gehäuse und mindestens eine in einem oberen Bereich des Gehäuses angebrachte Lichtquelle, die Licht eines vorbestimmten Wellenlängenbereichs emittiert.

### Stand der Technik

Fluoreszierende Substanzen können beispielsweise als Zusatz zu kosmetischen oder dermatologischen Substanzen sowie zu Hygieneprodukten als Markierungssubstanzen eingesetzt werden. Typische Anwendungsprodukte dieser Art sind zum Beispiel, Schutz-, Reinigungs- oder Pflegepräparate, wie zum Beispiel Hautschutzcremes, Feuchtigkeitscremes, Sonnencremes sowie rein medizinische Präparate. Darüber hinaus lassen sich fluoreszierende Substanzen auch als Zusatzstoff zu Hygienepräparaten verwenden und ermöglichen somit eine besonders einfache Form der Anwendungsüberprüfung, beispielsweise im Rahmen einer Schulung oder medizinischen Aufklärung. Dabei handelt es sich um das beispielsweise in der Dermatologie als Fluoreszenztechnik bekannte Verfahren. Es wird die spezifische Eigenschaft des fluoreszierenden Stoffes ausgenutzt, bei Bestrahlung durch Licht einer spezifischen Wellenlänge in einer charakteristischen Farbe aufzuleuchten. Da die fluoreszierende Substanz ansonsten bevorzugt farblos ist, können auf diese Weise für eine Schulung die gewünschte Visualisierungen besonders eindrucksvoll durchgeführt werden.

Um die Verteilung einer solchen fluoreszierenden Substanz beispielsweise auf den Händen einer Testperson sichtbar zu machen, sind aus dem Stand der Technik verschiedene Vorrichtungen bekannt. In der EP 0 913 681 wird beispielsweise eine Vorrichtung beschrieben, die ein aufwändiges Gehäuse mit entsprechenden Leuchtmitteln und seitlichen Öffnungen zum Einbringen eines zu untersuchenden Objekts aufweist, das ferner mit einer Kontrollöffnung zum Beobachten des untersuchten Gegenstands versehen ist. Bei einer derartigen Vorrichtung können besonders gute Voraussetzungen zum Sichtbarmachen der fluoreszierenden Substanz, insbesondere auf den Händen einer Testperson bereitgestellt werden. Nachteilig ist jedoch, dass die Vorrichtung aufgrund ihrer aufwändigen Gestaltung relativ unhandlich ist, sich nicht besonders platzsparend und praktisch und nicht repräsentativ deponieren lässt.

Die DE 102 47 605 A1 schlägt vor, eine zusammenfaltbare Händehygienefaltbox zu verwenden, die in einem aufgeklappten Zustand ein abgeschlossenes Gehäuse mit zwei Öffnungen für die Hände einer Testperson bildet und im zusammengeklappten Zustand platzsparend deponiert werden kann. Ein Nachteil der in dieser Offenlegungsschrift dargestellten Konstruktion ist jedoch ein relativ aufwändiges Verfahren zum Auseinander- bzw. Zusammenfalten der Faltbox. Insgesamt ist die Faltbox, die in der DE 102 47 605 offenbart wird, eine wenig repräsentative Vorrichtung zum Sichtbarmachen von fluoreszierenden Substanzen.

### Darstellung der Erfindung

Die Aufgabe der vorliegenden Erfindung liegt darin, eine dem oben genannten technischen Gebiet zugehörende Vorrichtung zu schaffen, die außerhalb des Betriebs, d.h. außerhalb ihrer Anwendung oder Benutzung, repräsentativ platzierbar und schnell, unkompliziert in einen betriebsbereiten Zustand versetzbar ist und bei der Verwendung die psychologischen und didaktischen Anforderungen besonders gut erfüllt.

Die Aufgabe wird durch die Merkmale des Anspruchs 1 gelöst. Vorteilhafte Weiterbildungen der Erfindung ergeben sich aus den Unteransprüchen.

In einer aufrechten Position der Vorrichtung kann die Vorrichtung in ihrer ersten Stellung beispielsweise als Werbemittel verwendet werden, indem sie aufrecht stehend in ein Regal, auf einem Tisch oder auf einer Ausstellungsplattform positioniert wird. Die Vorrichtung ist dabei allgemein schlank. Die Höhe der Vorrichtung ist größer als ihre Tiefe. In der ersten Stellung kann die Vorrichtung dabei nahezu beliebig, beispielsweise durch den Einsatz von Folien, beispielsweise so genannten 3D- oder Metall-Folien, mit denen die Seitenflächen sowie die Decke und der Boden des Gehäuses der Vorrichtung bedeckt sein können, dekoriert werden. Die verschiedenen Gestaltungsmöglichkeiten der Oberfläche des Gehäuses beinhalten dabei auch ein Bedrucken, ein Verschweißen von unter einer durchsichtigen Folienoberfläche abgedeckten und dort hinterlegten Drucken sowie Folientaschen zum Einschieben von einzelnen Drucken. Besonders bevorzugt kann die Vorrichtung in ihrer ersten Stellung durch ihre äußere Erscheinung den Eindruck eines herkömmlichen Aktenordners oder eines Werbeordners erwecken, da sich die Maße des Gehäuses besonders bevorzugt an denen eines solchen Aktenordners oder Werbeordners orientieren können. Es wird damit gewissermaßen ein Werbeordner zur Verfügung gestellt, der in seinem Inneren mit Elektronik ausgestattet ist.

In der zweiten Stellung des Gehäuses weist das Gehäuse genau eine seitliche Öffnung in einer der vier Seiten auf, die einen Zugang zum Inneren des Gehäuses der Vorrichtung darstellt. Durch diese Öffnung ist es möglich, dass ein Benutzer beispielsweise seine Hände oder andere zu untersuchende Objekte in das Innere des Gehäuses gelangen lässt, wobei erfindungsgemäß eine besonders einfache Ausgestaltung einer entsprechenden Vorrichtung zum visuellen Erkennen und/oder Darstellen der Verteilung von fluoreszierenden Substanzen geschaffen wird. Das Vorhandensein einer Öffnung für beide Hände einer Testperson ermöglicht es zudem, im Gegensatz zu einer Vorrichtung mit je einer Öffnung für eine Hand, dass beispielsweise Einreibetechniken der Testperson schon während der Ausführung im Gehäuse untersucht werden können. Zudem ist es somit möglich, Rechts- bzw. Linkshänder-spezifische Eigenarten bei den zu untersuchenden Handlungen zu untersuchen. Eine große Öffnung für beide Hände, die den Eindruck eines offenen Raums im Innern des Gehäuses vermittelt, ist außerdem für die Testperson akzeptabler, weil sie ihre Hände nicht in einen uneinsehbaren Raum stecken muss. Ferner kann ein Tutor die Testperson buchstäblich "an der Hand nehmen", indem er die Testperson bei der Anordnung oder Positionierung der Hände für die bestimmungsgemäße Durchführung eines Tests unterstützt.

Bei den lumineszierenden, insbesondere fluoreszierenden Substanzen kann es sich um einen Zusatz zu kosmetischen oder dermatologischen Präparaten oder auch um eine eigens für den Zweck der Analyse angewendete Testlotion, um eine wässrige oder alkoholische Flüssigkeit oder viskose Paste handeln, die zu Schulungszwecken oder zur Überprüfung einer Applikation von nahezu beliebigen Präparaten, insbesondere für Hygiene- oder Desinfektionsanwendungen, verwendet wird. Es kann hierbei einerseits die Applikation von Präparaten, die Desinfizierung, Handtrocknung, Verwendung von Papiertüchern, Infektionskontrolle oder ähnliches simuliert werden. Andererseits ist es auch möglich, das Vorhandensein und die Verteilung von lumineszierenden Substanzen, beispielsweise von Urin, Bakterien oder chininhaltigen Substanzen zu prüfen. Die Vorrichtung kann daher insbesondere im Bereich der Medizin, Pharmakologie, Lebensmittelindustrie/Gastronomie, Labortechnik, Sanitärreinigung, Schulung oder Kriminaltechnik Anwendung finden.

Der Wellenlängenbereich des Lichts aus der im oberen Bereich des Gehäuses angebrachten Lichtquelle liegt insbesondere im ultravioletten Bereich, erstreckt sich also vom kurzwelligen Rand des sichtbaren Lichtspektrums in Richtung kürzerer Wellenlängen, da hier gängige fluoreszierende Substanzen besonders gut erkennbar sind. Der obere Bereich des Gehäuses, in dem die Lichtquelle angebracht ist, ist dabei für die aufrecht stehende Position des Gehäuses definiert, in der das Gehäuse auf dem Gehäuseboden steht. Die Seiten des Gehäuses erstrecken sich dabei im Wesentlichen vertikal zwischen dem Gehäuseboden und dem oberen Bereich des Gehäuses und schließen mit der Gehäusedecke ab.

Dadurch, dass sich das Gehäuse leicht zwischen einer geschlossenen ersten Stellung und einer geöffneten zweiten Stellung umstellen lässt, ist es besonders einfach möglich, dass ein Benutzer, beispielsweise ein Schulungsleiter, eine beispielsweise in seinem Regal stehende Vorrichtung entfaltet, dabei die seitliche Öffnung freigibt und unmittelbar nach einem Anschließen einer elektrischen Stromquelle mit der Benutzung der erfindungsgemäßen Vorrichtung beginnen kann. Eine separate weitere Öffnung des Gehäuses, beispielsweise zum Betrachten der Testobjekte ist hierbei nicht nötig, da die eine Öffnung sowohl einen Zugang zum Inneren des Gehäuses freigibt als auch einen freien Blick in das Innere des Gehäuses ermöglicht.

In einer weiteren Ausführungsform ist die seitliche Öffnung durch ein Teilelement zumindest teilweise unterbrochen. Das Unterbrechen der genau einen seitlichen Öffnung in dem Gehäuse der Vorrichtung durch ein einzelnes Teilelement, beispielsweise durch eine Stütze oder eine Markierung, kann je nach Ausführungsform des Gehäuses von Vorteil sein. Beispielsweise können durch das Teilelement definierte Gebiete für je eine zu untersuchende Hand zur Verfügung gestellt werden, so dass die zu untersuchenden Hände voneinander entfernt sind und sich beispielsweise nicht gegenseitig abschatten können. Im Fall eines horizontal ausgerichteten Teilelements kann dieses auch als Markierung dienen, unterhalb derer die Testperson ihre Hände in die Öffnung schieben soll. Schließlich ist es auch möglich, mehrere Teilelemente, beispielsweise ein horizontales und ein vertikales Teilelement vorzusehen. Es wird jedoch bevorzugt, dass die Öffnung nicht durch ein Teilelement unterbrochen ist.

Mit Vorteil weist das Gehäuse eine aufklappbare, bevorzugt zumindest zweiteilig aufklappbare, besonders bevorzugt dreiteilig aufklappbare Seitenwand auf, die in der zweiten Stellung über die Grundfläche des Gehäuses in der ersten Stellung hinausragend angeordnet ist. Durch das Aufklappen der Seitenwand kann die Öffnung des Gehäuses freigegeben werden. Hierzu ist es nicht nötig, weitere Elemente des Gehäuses durch einen aufwändigen Faltprozess zu verändern, sondern ein simples Aufklappen einer Seitenwand ist zum Freigeben der seitlichen Öffnung ausreichend.

In der bevorzugten Ausführungsform, in der die Seitenwand zumindest zweiteilig aufklappbar ist, kann jeder Teil der Seitenwand darüber hinaus als zusätzliche Stütze fungieren, wenn die Teile der Seitenwand beispielsweise um eine im Wesentlichen vertikale Achse nach außen geschwenkt werden und damit über die Grundfläche des Gehäuses in der ersten Stellung hinausragen.

In der weiter besonders bevorzugten Ausführungsform eines Gehäuses mit einer dreiteiligen aufklappbaren Seitenwand lässt sich die Seitenwand einerseits um jeweils eine im Wesentlichen vertikal ausgerichtete Schwenkachse nach außen schwenken und bildet damit eine in Blickrichtung auf die Öffnung des Gehäuses hin linke und rechte Seitenwand und umfasst darüber hinaus einen dritten Teil, der beispielsweise um eine im Wesentlichen horizontal ausgerichtete Schwenkachse nach unten klappbar ist und damit einen erweiterten Bodenbereich des Gehäuses in der geöffneten zweiten Stellung darstellt.

Somit kann in der geöffneten Stellung eine Erweiterung des teilweise umschlossenen Bereichs des Gehäuses auf besonders einfache Weise erreicht werden, indem eine Seitenwand bevorzugt zweiteilig nach außen geschwenkt und dadurch zu einer Verlängerung der beiden der Seitenwand in ihrer geschlossenen Position benachbarten Seitenwände wird. Besonders bevorzugt wird in der dreiteiligen Ausführungsform ein zusätzlich ausklappbarer Teil der Seitenwand um eine im Wesentlichen horizontale Achse nach unten geschwenkt und bildet im ausgeklappten Zustand den erweiterten Bodenteil. Die Seitenwand ist ein Teil des Gehäuses und verschließt das Gehäuse in der ersten Stellung. Zu der Seitenwand gehören bevorzugt zwei in der ersten und der zweiten Stellung im Wesentlichen vertikal ausgerichtete Seitenteile und besonders bevorzugt der weitere Teil, der in der ersten Stellung zu einer Seitenwand hochgeklappt ist.

Mit Vorteil weist die Vorrichtung eine Fixierungseinrichtung, insbesondere einen oder mehrere Magnete und/oder einen oder mehrere Klettverschlüsse zum reversiblen Fixieren des Gehäuses, insbesondere der Seitenwand, in der ersten Stellung auf. Die Fixierungseinrichtung zum reversiblen Fixieren des Gehäuses in der ersten Stellung hat den Vorteil, dass das Gehäuse in der ersten Stellung sicher verschlossen ist und sowohl das Innere des Gehäuses vor Verschmutzung oder Beschädigung oder anderen äußeren Einwirkungen schützt als auch die Umgebung des Gehäuses gegenüber schädlichen Einflüssen, beispielsweise durch eine zerborstene Lichtquelle oder ähnliches aus dem Innern des Gehäuses bewahrt. Bevorzugt handelt es sich bei der Fixierungseinrichtung um einen oder mehrere Magnete und/oder einen oder mehrere Klettverschlüsse, daneben sind auch Schlaufen, Spangen, Druck- oder Drehknöpfe als Fixierungseinrichtungen möglich. Diese Fixierungseinrichtungen haben den Vorteil, dass sie einerseits äußerst einfach zu schließen und zu öffnen sind und andererseits auch optisch problemlos in das Gesamtbild des Gehäuses einbindbar sind.

Die Öffnung ist dazu geeignet, gleichzeitig zwei Hände eines Benutzers aufzunehmen. Die Öffnung ist folglich so dimensioniert, dass zwei Hände eines Benutzers bequem nebeneinander in der Öffnung Platz finden. Somit ist auch ein vergleichendes Sichtbarmachen der Verteilung von einer fluoreszierenden Substanz auf den Händen, beispielsweise zur Indikation der Gründlichkeit eines Händewasch- oder Desinfektionsvorgangs, möglich.

Ferner umfasst die Lichtquelle zumindest eine, bevorzugt zwei UV-Röhren und/oder mehrere Leuchtdioden, die in einem im oberen Bereich des Gehäuses angeordneten Elektronikgehäuse aufgenommen sind. Die UV-Röhren und/oder mehreren Leuchtdioden sind dazu geeignet, Licht des vorbestimmten Wellenlängenbereichs zu emittieren, um die Verteilung von fluoreszierenden Substanzen darzustellen. Hierbei handelt es sich bevorzugt um Licht des ultravioletten Spektralbereichs. Grundsätzlich ist, in Abhängigkeit von der anzuregenden fluoreszierenden Substanz, elektromagnetische Strahlung sämtlicher Wellenlängen zum Anregen der fluoreszierenden Substanz und dem damit verbundenen Sichtbarmachen möglich. Bei bevorzugten fluoreszierenden Substanzen erfolgt die Anregung jedoch durch Licht des ultravioletten Spektralbereichs.

Das Elektronikgehäuse umfasst neben der Lichtquelle Elektrik- und Elektronikbauteile, die in oder an dem Elektronikgehäuse untergebracht sind. Hierzu gehören unter anderem ein Steckeranschluss, ein USB-Eingang, ein AN/AUS-Schalter, ein Starter für eventuell vorhandene Röhren sowie eine Steuerungselektronik für den Betrieb der Lichtquelle. Das Elektronikgehäuse, das im oberen Bereich des Gehäuses angeordnet ist, bildet bevorzugt zudem das Dach, also die obere Begrenzung des Gehäuses.

Bevorzugt ist das Elektronikgehäuse an drei Seiten des Gehäuses an dem Gehäuse befestigt, nämlich auf zwei gegenüberliegenden Seiten des Gehäuses formschlüssig und auf einer dritten Seite des Gehäuses kraftschlüssig. Beispielsweise kann das Elektronikgehäuse an zwei Seitenwänden, die jeweils nicht öffnungsfähig sind, durch eine oder mehrere Schrauben oder Nieten oder ähnliche formschlüssige Befestigungselemente befestigt sein und auf einer dritten Seitenwand, die ebenfalls nicht öffnungsfähig ist, kraftschlüssig, beispielsweise durch einen Klebestreifen befestigt sein. Die vierte noch verbleibende Seitenwand ist bevorzugt öffnungsfähig und dient nicht der Befestigung des Elektronikgehäuses.

Es ist grundsätzlich auch möglich, die Befestigung des Elektronikgehäuses an dem Gehäuse anderweitig vorzunehmen. Insbesondere ist es möglich, dass das Elektronikgehäuse an der dritten Seite des Gehäuses nur anliegt und durch die Befestigungen an den zwei ersten Seitenwänden vollständig sicher gehalten wird. Daneben ist auch eine formschlüssige Befestigung des Elektronikgehäuses an der dritten Seite möglich, beispielsweise durch eine entsprechende Ausformung des Elektronikgehäuses und/oder des Gehäuses der Vorrichtung sowie eine Befestigung des Elektronikgehäuses an den zwei ersten Seiten des Gehäuses durch Kraftschluss, beispielsweise Klebstoff oder ähnliche Befestigungsmittel.

Erfindungsgemäß weist die Vorrichtung eine Höhe von zwischen 25 cm und 35 cm und, in ihrer ersten Stellung, eine Tiefe von zwischen 5 cm und 10 cm auf. Die Tiefe erstreckt sich dabei in einer Richtung senkrecht zur Ausrichtung der aufklappbaren und damit öffnungsfähigen Seitenwand. In der ersten Stellung hat die Vorrichtung damit bevorzugt die Maße eines herkömmlichen Aktenordners, was eine besonders einfache Anordnung der Vorrichtung, beispielsweise in einem Bücherregal, nahelegt. In der ersten Stellung wird demnach die Grundfläche der Vorrichtung einerseits durch die Breite des Gehäuses entlang der aufklappbaren Seitenwand und der rückseitigen Seitenwand und andererseits durch die Tiefe senkrecht zur aufklappbaren Seitenwand von bevorzugt zwischen 5 cm und 10 cm definiert. Da in der geöffneten zweiten Stellung die Seitenwand und bevorzugt ein ausklappbarer Bodenteil über die Grundfläche der Vorrichtung in der ersten Stellung hinausragt, ist die Grundfläche der Vorrichtung in der zweiten Stellung einerseits durch die Breite der Vorrichtung und andererseits durch eine erweiterte Tiefe, die neben der vorgenannten Tiefe von zwischen 5 cm und 10 cm noch die jeweilige Länge des ausgeklappten Teils der Seitenwand und/oder des erweiterten Bodenteils umfasst.

Mit Vorteil ist die Lichtquelle eingerichtet, Licht in ein definiertes, unterhalb der Lichtquelle liegendes Raumsegment zu emittieren, das bevorzugt kleiner als ein Viertelzylinder, besonders bevorzugt kleiner als ein Achtelzylinder ist. Die Zylinderachse des Viertelzylinders bzw. des Achtelzylinders verläuft dabei bevorzugt horizontal, beispielsweise entlang der Lichtquelle, beispielsweise der UV-Röhre; eine seitliche Begrenzung des Zylindersegments wird durch die rückseitige Seitenwand gebildet, die der öffnungsfähigen Seitenwand gegenüberliegt; und die vordere Begrenzung des Zylindersegments ist durch die Ausgestaltung der Lichtquelle bzw. der Anordnung der Lichtquelle, insbesondere in dem Elektronikgehäuse, definiert. Hierbei kann unter anderem beispielsweise die Vorderwand des Elektronikgehäuses als Abschattung fungieren, die das Licht der Lichtquelle daran hindert, in einen ungewünschten Raumbereich, beispielsweise in Richtung des Gesichts einer Testperson oder dergleichen, zu strahlen, was zur hohen Sicherheit der Vorrichtung beiträgt. Damit wird ein definierter Abstrahlwinkel geschaffen, innerhalb dessen sich das Licht des vorbestimmten Wellenlängenbereichs frei ausbreitet und der folglich zum visuellen Erkennen und/oder Darstellen der Verteilung von fluoreszierenden Substanzen geeignet ist. Da sich das Licht in diesem Fall auch durch die Öffnung vor das Innere des Gehäuses ausbreitet, ist es zudem möglich, dass eine Testperson ihre Hände auch vor der Öffnung zum Sichtbarmachen einer fluoreszierenden Substanz anordnen kann, was die Sicherheit des Geräts auf Grund einer niedrigeren Strahlungsintensität vor der Öffnung erhöht.

Eventuell stattfindende Reflexion durch die seitlichen Seitenwände oder die rückseitige Seitenwand sowie den Boden wird hierbei zur Definition des Raumsegments nicht weiter berücksichtigt. Es ist also möglich, dass trotz des definierten Raumsegments, in das die Lichtquelle das Licht emittiert, auch ein visuelles Erkennen und/oder Darstellen der Verteilung von fluoreszierenden Substanzen außerhalb des definierten Raumsegments möglich ist, nämlich aufgrund der oben erwähnten Reflexion. Einerseits, um die vorgenannte Reflexion zu minimieren, andererseits zur besseren optischen Wirkung einer angeregten fluoreszierenden Substanz, ist dass Innere des Gehäuses bevorzugt schwarz oder in dunklen Farben ausgeführt. Es kann auch teilweise schwarz oder in dunklen Farben ausgeführt sein. Durch die dunklen Farben des Inneren des Gehäuses kann ein starker Kontrast zu den Leuchteffekten im Innern des Gehäuses hergestellt werden.

Es ist jedoch auch möglich, dass spiegelnde Flächen im Innern des Gehäuses von Vorteil sind, beispielsweise, wenn zu untersuchende Hände von mehreren Seiten beleuchtet werden sollen. In diesem Fall ist es bevorzugt, das Innere des Gehäuses beispielsweise mit Spiegelfolie auszukleiden und damit eine gleichmäßige, mehrseitige Bestrahlung der Hände zu ermöglichen. Bei der Verwendung der Vorrichtung zum visuellen Erkennen der Verteilung von Substanzen auf dunkler Haut kann vorgesehen werden, eine zusätzliche Lichtquelle, beispielsweise eine Schreibtischlampe oder dergleichen zum Aufhellen des Innenraums des Gehäuses einzusetzen. Zusätzlich kann eine weiße Oberfläche, beispielsweise als ein optischer Boden des Gehäuses aufgelegt werden, um den Kontrast zwischen dem Innern des Gehäuses und der zu bewertenden Körperpartie zu erhöhen.

Mit Vorteil weist die Vorrichtung einen ausgezeichneten Raumbereich zum Positionieren der zu untersuchenden Objekte, insbesondere der Hände eines Benutzers, auf. Die Auszeichnung des Raumbereichs kann beispielsweise durch Markierungen an dem Gehäuse der Vorrichtung erfolgen, andererseits ist es möglich, das Gehäuse derart zu gestalten, dass die zu untersuchenden Objekte, beispielsweise die Hände eines Benutzers, automatisch in dem zur Untersuchung günstigen Raumbereich angeordnet sind.

Die Vorrichtung weist eine Befestigungseinrichtung für eine Foto- oder Filmkamera, welche die zu untersuchenden Objekte im Innern des Gehäuses filmt oder fotografiert und damit eine besonders gute Möglichkeit
der Dokumentation des Untersuchungsergebnisses liefert, auf. Hierdurch wird zudem die
Möglichkeit einer Dokumentation sowie einer Vorführung der Untersuchung vor einer größeren Personengruppe z.B. auf einem TV-Bildschirm oder einer Videoleinwand ermöglicht.

In einer bevorzugten Ausführungsform weist das öffnungsfähige Gehäuse Ausnehmungen zum Aufnehmen und/oder Betätigen eines Betätigungselements, insbesondere eines EIN/AUS-Schalters oder eines Umschalters, beispielsweise zum Umschalten zwischen verschiedenen Beleuchtungsmodi (z.B. UV-Licht- und Weißlichtmodus oder hellere und dunklere Beleuchtung) der Lichtguelle auf, und/oder zum Durchführen einer elektrischen Stromversorgungs- oder Datenleitung, beispielsweise eines USB-Anschlusskabels, auf. In dieser bevorzugten Ausführungsform ist es also möglich, dass kein weiteres Betätigungselement und keine elektrische Anschlusseinrichtung auf der Außenseite des Gehäuses angeordnet sind. Vielmehr können alle Betätigungselemente und elektrische Anschlusseinrichtungen im Innern des Gehäuses bzw. in den Wänden des Gehäuses aufgenommen sein. Dieses Merkmal ermöglicht eine besonders repräsentative Ausgestaltung des Gehäuses der Vorrichtung in dem geschlossenen ersten Zustand.

Mit Vorteil weist eine Seitenwand oder ein Bodenelement des Gehäuses eine Befestigungseinrichtung für einen Gegenstand, insbesondere einen Behälter auf. Die bevorzugt im Innern des Gehäuses angebrachte Befestigung ermöglicht es, dass beispielsweise für die Verwendung der Vorrichtung notwendige Gegenstände, wie beispielsweise ein Anschlusskabel, ein Stromadapter, eine Bedienungsanleitung, Schulungsunterlagen, eine Taschenlampe, die beispielsweise eine UV-Lichtquelle in Form von Leuchtdioden aufweisen kann und mit der beispielsweise Reinigungserfolge auf externen Oberflächen untersucht werden können, ein USB-Anschlusskabel, eine Halterungseinrichtung, Batterien oder Akkus für einen netzstromunabhängigen Betrieb, Behälter für fluoreszierende Substanzen, Applikationswerkzeuge und vieles mehr im Innern des Gehäuses untergebracht werden können. In einer besonders bevorzugten Ausführungsform sind die genannten Gegenstände zumindest teilweise in einem gemeinsamen Behälter aufgenommen, der bevorzugt quaderförmig ausgebildet ist. Durch entsprechende Befestigungseinrichtungen kann der Behälter sicher an dem Gehäuse befestigt und daher im Innern des Gehäuses aufgenommen sein.

Weitere bevorzugte Ausführungsformen ergeben sich aus der nachfolgenden Figurenbeschreibung sowie der Gesamtheit der Ansprüche.

### Kurze Figurenbeschreibung

- Fig. 1: zeigt eine Ausführungsform in der geschlossenen ersten Stellung;
- Fig. 2: zeigt die Ausführungsform aus Fig. 1 in der geöffneten zweiten Stellung.

### Wege zur Ausführung der Erfindung

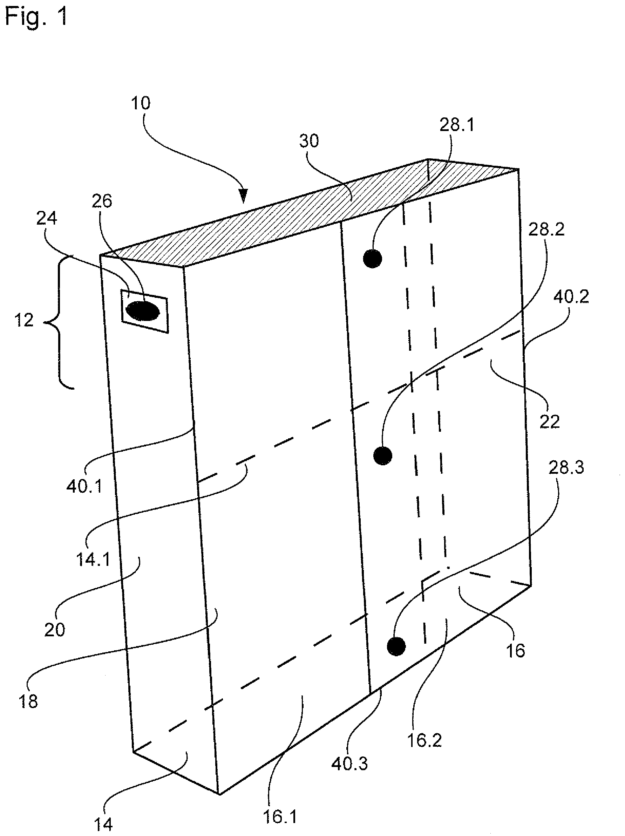

Fig. 1 zeigt eine Ausführungsform der Vorrichtung in einer geschlossenen ersten Stellung. Die Vorrichtung weist ein quaderförmiges Gehäuse 10 auf, das im Wesentlichen den Maßen und Proportionen eines herkömmlichen Aktenordners entspricht. Das Gehäuse 10 steht in der in Fig. 1 gezeigten Situation aufrecht, d.h. auf seiner Bodenfläche 14. Die Bodenfläche 14 ist dabei, wie in Fig. 1 deutlich zu erkennen ist, kleiner als zumindest zwei Seitenwände 16, 18 des Gehäuses 10. Die zwei Seitenwände 16, 18 definieren dabei die Breite des Gehäuses 10. Neben den zwei genannten Seitenwänden 16, 18 weist das Gehäuse zwei weitere Seitenwände 20, 22 auf, die zusammen mit den zwei erstgenannten Seitenwänden 16, 18 eine Seitenwandanordnung mit einem rechteckigen Grundriss bilden und sich in Richtung der Tiefe des Gehäuses 10 erstrecken. In Fig. 1 ist aus Gründen der besseren Darstellbarkeit ebenfalls gezeigt, wie die verdeckten Kanten zwischen der Bodenfläche 14 und den Seitenwänden 18, 22 sowie zwischen den Seitenwänden 18, 22 verlaufen.

Die Seitenwand 16 lässt sich in der in Fig. 1 dargestellten Ausführungsform zweiteilig öffnen, wobei in der geschlossenen ersten Stellung zwei Teile 16.1, 16.2 der Seitenwand 16 aufeinander gefaltet sind und damit das Innere des Gehäuses 10 abschließen. Die beiden Teilseitenwände 16.1, 16.2 sind dabei durch drei Magnetverschlüsse 28.1 bis 28.3, die entlang eines Überlappungsbereichs zwischen den beiden Teilseitenwänden 16.1, 16.2 angeordnet sind, in der geschlossenen ersten Stellung reversibel fixiert.

Im oberen Bereich 12 des Gehäuses 10 ist ein Elektronikgehäuse 30 angeordnet, das in der geschlossenen ersten Stellung gleichzeitig eine Decke des Gehäuses 10 bildet. Das Elektronikgehäuse 30 umfasst dabei unter anderem auch die nicht dargestellte Lichtquelle zum Sichtbarmachen einer fluoreszierenden Substanz. Im oberen Bereich 12 des Gehäuses 10 kann in einer von den Figuren abweichenden Ausführungsform auch ein Halte- oder Traggriff angebracht sein, der einen Transport des Gehäuses erleichtert.

Das Gehäuse 10 weist an seiner links von der öffnungsfähigen Seitenwand 16 gelegenen Seitenwand 20 eine Ausnehmung 24 auf, in der ein AN/AUS-Schaltknopf 26 aufgenommen ist, mit dem die Lichtquelle im Innern des Elektronikgehäuses 30 ein- und ausschaltbar ist.

In Fig. 1 ist darüber hinaus angedeutet, dass die Bodenfläche 14 ein weiteres, in der geöffneten zweiten Stellung ausklappbares erweitertes Bodenflächenelement 14.1 aufweist, das in der geschlossenen ersten Stellung einen Teil der Seitenwand 16 bildet. Fig. 1 zeigt dabei eine mögliche Endkante des erweiterten Bodenelements 14.1 in der geschlossenen ersten Stellung. Dieses Bodenflächenelement 14.1 kann dabei in der geschlossenen ersten Stellung ebenfalls durch zumindest einen der Magneten 28.1 bis 28.3 in der aufrechten Position fixiert werden.

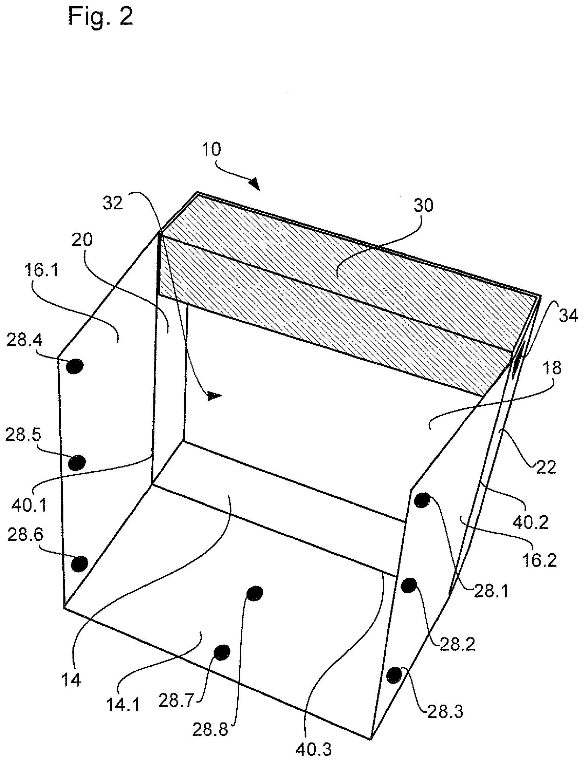

Während eines Aufklappens des Gehäuses 10 von der geschlossenen ersten Stellung aus Fig. 1 in Richtung der geöffneten zweiten Stellung aus Fig. 2 werden zunächst die Teilseitenwände 16.1, 16.2 entlang jeweils einer Schwenkachse 40.1, 40.2, die beispielsweise durch ein Foliengelenk realisiert sein kann, ausgeschwenkt. Dies erfolgt nach einem anfänglichen Überwinden der Kraft, die durch die Magnete 28.1 bis 28.3 auf die Teilseitenwände 16.1, 16.2 wirkt. Nachdem die Teilseitenwände 16.1, 16.2 in die geöffnete Stellung umgestellt sind, die in Fig. 2 zu sehen ist, lässt sich das erweiterte Bodenelement 14.1 entlang der Schwenkachse 40.3, die beispielsweise ebenfalls durch ein Foliengelenk gebildet sein kann, nach unten schwenken, wodurch die Bodenfläche in der zweiten Stellung vergrößert werden kann.

Fig. 2 zeigt das Gehäuse 10 aus Fig. 1 in der geöffneten zweiten Stellung, wobei die geöffneten Teilseitenwände 16.1, 16.2 sowie das geöffnete zusätzliche Bodenelement 14.1 eine Öffnung 32 zum Innern des Gehäuses 10 freigeben. Das Bodenelement 14.1 dient dabei unter anderem dazu, den Untergrund, beispielsweise einen Tisch, auf dem sich das Gehäuse befindet, vor schädlicher UV-Strahlung zu schützen. Fig. 2 zeigt deutlich das Elektronikgehäuse 30 im oberen Teil des Gehäuses 10, in dem auch die Lichtquelle, beispielsweise zwei UV-Röhren, untergebracht sind. Die in Fig. 1 nicht von außen sichtbare Seitenwand 22 weist einen elektrischen Anschluss 34 auf, der in der hier gezeigten Ausführungsform zum anschließen der Lichtquelle an ein externes Stromnetz dient. Der Anschluss 34 ist dabei ebenfalls, wie der AN/AUS-Schalter 26, in einer Ausnehmung in der Seitenwand 22, die zur Ausnehmung 24 in der Seitenwand 20 analog ist, aufgenommen. Neben den Magneten 28.1 bis 28.3, die schon in Fig. 1 dargestellt sind, zeigt Fig. 2 auch die weiteren Magneten 28.4 bis 28.6, die sich auf der Teilseitenwand 16.1 befinden und als Gegenpol zu den Magneten 28.1 bis 28.3 in der Teilseitenwand 16.2 fungieren. Darüber hinaus zeigt Fig. 2 weitere Magnete 28.7, 28.8, die sich auf dem erweiterten Bodenflächenelement 14.1 befinden und dieses in der geschlossenen ersten Stellung aus Fig. 1 in der aufrechten Position an den Teilseitenwänden 16.1, 16.2 halten.

Die Seitenwände sowie die Bodenfläche des Gehäuses sind in der hier dargestellten Ausführungsform aus Karton oder Pappe mit darauf aufgebrachten Folien ausgeführt. Statt des Kartons sind auch andere Materialien wie beispielsweise Kunststoff oder ein Glasfaserverbund denkbar und die Folie kann beispielsweise durch moderne Druckverfahren gestaltet werden. Ein Beispiel eines solchen Druckverfahrens ist der sogenannte Offset und der UV-Offsetdruck.

Die Seitenwände und die Bodenfläche sind bevorzugt aus einem durchgehenden Stück hergestellt. Hierbei handelt es sich bevorzugt um ein faltbares Muster von mit Folie beschichteten Karton oder Kunststoffelementen. Die Folie ist bevorzugt beidseitig auf den Karton- oder Kunststoffelementen aufgetragen und verläuft durchgehend auf allen Karton- oder Kunststoffelementen. An den zu faltenden Übergangsstellen zwischen verschiedenen Karton- oder Kunststoffelementen bildet die Folie jeweils ein Foliengelenk, mit dem einerseits die einzelnen Elemente miteinander verbunden und andererseits gegeneinander schwenkbar sind. Für die Folie kann neben einer herkömmlichen Kunststofffolie auch eine Metallfolie oder eine der oben genannten Folienausführungen verwendet werden. Ferner sind andere Gelenke, wie Knick- oder Faltgelenke sowie Scharniere denkbar.

## Patentansprüche

1. Vorrichtung zum visuellen Erkennen und/oder Darstellen der Verteilung von lumineszierenden, insbesondere fluoreszierenden Substanzen auf den beiden Händen eines Benutzers, nämlich einer Testperson, umfassend
ein seitlich öffnungsfähiges Gehäuse (10) und mindestens eine in einem oberen Bereich (12) des Gehäuses (10) angebrachte Lichtquelle, die Licht eines vorbestimmten Wellenlängenbereichs im ultravioletten Bereich emittiert,
wobei das Gehäuse (10) zwischen einer geschlossenen ersten Stellung zum Deponieren der Vorrichtung außerhalb ihres Betriebs, d.h. außerhalb ihrer Anwendung oder Benutzung, und einer geöffneten zweiten Stellung zum Betreiben der Vorrichtung, d.h. zum visuellen Erkennen und/oder Darstellen der Verteilung der lumineszierenden, insbesondere fluoreszierenden Substanzen auf den beiden Händen der Testperson, umstellbar ist, wobei die Fläche des Gehäusebodens (14) in der ersten Stellung kleiner ist als zumindest zwei Seitenflächen (16, 18) des Gehäuses (10),
**dadurch gekennzeichnet, dass** das Gehäuse (10) in der zweiten Stellung genau eine seitliche Öffnung (32) in einer der vier Seiten (16, 18, 20, 22) zu seinem Inneren aufweist, wobei die Öffnung (32) geeignet ist, gleichzeitig mindestens zwei Hände der Testperson aufzunehmen,
wobei die Lichtquelle zumindest eine UV-Röhre und/oder mehrere Leuchtdioden umfasst, die in einem im oberen Bereich (12) des Gehäuses (10) angeordneten Elektronikgehäuse (30) aufgenommen sind,
wobei die Vorrichtung eine Höhe von zwischen 25 cm und 35 cm und in der ersten Stellung eine Tiefe von zwischen 5 cm und 10 cm aufweist, und
wobei die Vorrichtung eine Befestigungseinrichtung für eine Fotokamera oder Filmkamera zum Fotografieren oder Filmen der Hände der Testperson im Innern des Gehäuses aufweist.

2. Vorrichtung nach Anspruch 1, **dadurch gekennzeichnet, dass** die seitliche Öffnung (32) durch ein Teilelement zumindest teilweise unterbrochen ist.

3. Vorrichtung nach einem der Ansprüche 1 bis 2, **dadurch gekennzeichnet, dass** das Gehäuse (10) eine aufklappbare, bevorzugt zumindest zweiteilig aufklappbare, besonders bevorzugt dreiteilig aufklappbare Seitenwand (16, 16.1, 16.2) aufweist, die in der zweiten Stellung über die Fläche des Gehäusebodens (14) in der ersten Stellung hinausragend angeordnet ist.

4. Vorrichtung nach einem der Ansprüche 1 bis 3, **dadurch gekennzeichnet, dass** sie eine Fixierungseinrichtung (28.1-28.8), insbesondere einen oder mehrere Magnete und/oder einen oder mehrere Klettverschlüsse zum reversiblen Fixieren des Gehäuses (10), insbesondere der Seitenwand (16), in der ersten Stellung aufweist.

5. Vorrichtung nach einem der Ansprüche 1 bis 4, **dadurch gekennzeichnet, dass** das Elektronikgehäuse (30) auf zwei gegenüberliegenden Seiten des Gehäuses (10) formschlüssig und auf einer dritten Seite des Gehäuses (10) kraftschlüssig an dem Gehäuse (10) befestigt ist.

6. Vorrichtung nach einem der Ansprüche 1 bis 5, **dadurch gekennzeichnet, dass** die Lichtquelle eingerichtet ist, Licht in ein definiertes, unterhalb der Lichtquelle liegendes Raumsegment zu emittieren, das bevorzugt kleiner als ein Viertelzylindersegment, besonders bevorzugt kleiner als ein Achtelzylindersegment ist, wobei die Zylinderachse bevorzugt entlang der Lichtquelle verläuft.

7. Vorrichtung nach einem der Ansprüche 1 bis 6, **dadurch gekennzeichnet, dass** sie einen ausgezeichneten Raumbereich zum Positionieren der Hände der Testperson aufweist.

8. Vorrichtung nach einem der Ansprüche 1 bis 7, **dadurch gekennzeichnet, dass** das Gehäuse Ausnehmungen (24, 34) zum Aufnehmen und/oder Betätigen eines Betätigungselements, insbesondere eines Ein-/Aus-Schalters (26) oder eines Umschalters, und/oder zum Durchführen einer elektrischen Stromversorgungs- oder Datenleitung aufweist.

9. Vorrichtung nach einem der Ansprüche 1 bis 8, **dadurch gekennzeichnet, dass** eine Seitenwand (16, 16.1, 16.2, 18, 20, 33) oder ein Element des Gehäusebodens (14, 14.1) eine Befestigungseinrichtung für einen Gegenstand, insbesondere einen Behälter aufweist.

## Claims

1. Device for visual recognition of the distribution of luminescent, in particular fluorescent substances on the two hands of a user, namely a test person, comprising
a laterally openable housing (10) and
at least one light source attached in an upper region (12) of the housing (10) which emits light of a predetermined wavelength range within the ultraviolet range,
wherein the housing (10) can be switched between a closed first position for depositing the device outside of its operation, i.e. outside of its application or use, and an opened second position for operation of the device, i.e. for visual recognition and/or representation of the distribution of luminescent, in particular fluorescent substances on the two hands of the test person, wherein the surface area of the housing base (14) in the first position is smaller than at least two side surfaces (16, 18) of the housing (10),
**characterised in that** the housing (10) has, in the second position, exactly one lateral opening (32) to its interior in one of the four sides (16, 18, 20, 22), the opening (32) being suitable for accommodating at least two hands of the test person simultaneously,
wherein the light source comprises at least one UV tube and/or a plurality of LEDs which are housed in an electronics housing (30) arranged in an upper region (12) of the housing (10),
wherein the device has a height of between 25 cm and 35 cm and, in the first position, a depth of between 5 cm and 10 cm, and wherein the device has a fastening device for a photo camera or film camera for photographing or filming the hands of the test person within the interior of the housing.

2. Device according to claim 1, **characterised in that** the lateral opening (32) is at least partially interrupted by a dividing element.

3. Device according to one of the claims 1 to 2, **characterised in that** the housing (10) has an unfoldable, preferably at least two-part unfoldable, particularly preferably three-part unfoldable side wall (16, 16.1, 16.2) which, in the second position, is arranged so as to project above the surface of the housing base (14) in the first position.

4. Device according to one of the claims 1 to 3, **characterised in that** it has a fixing device (28.1-28.8), in particular one or more magnets and/or one or more Velcro closures, for reversible fixing of the housing (10), in particular the side wall (16), in the first position.

5. Device according to one of the claims 1 to 4, **characterised in that** the electronics housing (30) is fastened to the housing (10) in a form-fitting manner on two opposite sides of the housing (10) and in a force-locking manner on a third side of the housing (10).

6. Device according to one of the claims 1 to 5, **characterised in that** the light source is configured to emit light in a defined spatial segment located below the light source which is preferably smaller than a quarter cylinder segment, particularly preferably smaller than an eighth cylinder segment, wherein the cylinder axis preferably runs along the light source.

7. Device according to one of the claims 1 to 6, **characterised in that** it has an outstanding spatial region for positioning the hands of the test person.

8. Device according to one of the claims 1 to 7, **characterised in that** the housing has recesses (24, 34) for mounting and/or operating an operating element, in particular an on/off switch (26) or a toggle switch, and/or for passing through an electrical power supply or data cable.

9. Device according to one of the claims 1 to 8, **characterised in that** a side wall (16, 16.1, 16.2, 18, 20, 33) or an element of the housing base (14, 14.1) has a fastening device for an object, in particular a container.

## Revendications

1. Dispositif pour détecter et/ou représenter visuellement la répartition de substances luminescentes, en particulier fluorescentes, sur les deux mains d'un utilisateur, à savoir un sujet, comprenant
un boîtier (10) pouvant être ouvert latéralement et au moins une source de lumière montée dans une zone supérieure (12) du boîtier (10), qui émet de la lumière d'une gamme de longueurs d'onde prédéterminée dans le domaine de l'ultraviolet,
le boîtier (10) étant mobile entre une première position fermée pour le dépôt du dispositif en dehors de son fonctionnement, c'est-à-dire en dehors de son application ou de son utilisation, et une deuxième position ouverte pour le fonctionnement du dispositif, c'est-à-dire pour la reconnaissance et/ou la représentation visuelle de la répartition des substances luminescentes, en particulier fluorescentes, sur les deux mains du sujet, la surface du fond de boîtier (14) étant, dans la première position, plus petite qu'au moins deux surfaces latérales (16, 18) du boîtier (10),
**caractérisé en ce que** le boîtier (10) présente, dans la deuxième position, exactement une ouverture latérale (32) dans l'un des quatre côtés (16, 18, 20, 22) vers l'intérieur de celui-ci, l'ouverture (32) étant adaptée pour recevoir simultanément au moins deux mains du sujet,
la source de lumière comprenant au moins un tube UV et/ou plusieurs diodes électroluminescentes, qui sont logées dans un boîtier électronique (30) disposé dans la zone supérieure (12) du boîtier (10),
le dispositif présentant une hauteur comprise entre 25 cm et 35 cm et dans la première position une profondeur comprise entre 5 cm et 10 cm, et
le dispositif présentant un dispositif de fixation pour un appareil photo ou une caméra cinématographique destiné(e) à photographier ou filmer les mains du sujet à l'intérieur du boîtier.

2. Dispositif selon la revendication 1, **caractérisé en ce que** l'ouverture latérale (32) est au moins partiellement interrompue par un élément partiel.

3. Dispositif selon l'une quelconque des revendications 1 à 2, **caractérisé en ce que** le boîtier (10) présente une paroi latérale (16, 16.1, 16.2) relevable, de préférence au moins en deux parties, de manière particulièrement préférée en trois parties, et qui, dans la deuxième position, est disposée de manière à dépasser au-dessus de la surface du fond de boîtier (14) dans la première position.

4. Dispositif selon l'une quelconque des revendications 1 à 3, **caractérisé en ce qu'**il présente un dispositif de fixation (28.1-28.8), en particulier un ou plusieurs aimants et/ou une ou plusieurs fermetures auto-agrippantes pour la fixation réversible du boîtier (10), en particulier de la paroi latérale (16), dans la première position.

5. Dispositif selon l'une quelconque des revendications 1 à 4, **caractérisé en ce que** le boîtier électronique (30) est fixé au boîtier (10) par complémentarité de forme sur deux côtés opposés du boîtier (10) et par complémentarité de force sur un troisième côté du boîtier (10).

6. Dispositif selon l'une quelconque des revendications 1 à 5, **caractérisé en ce que** la source de lumière est agencée pour émettre de la lumière dans un segment spatial défini situé sous la source de lumière, qui est de préférence plus petit qu'un segment de quart de cylindre, de manière particulièrement préférée plus petit qu'un segment de huitième de cylindre, l'axe du cylindre s'étendant de préférence le long de la source de lumière.

7. Dispositif selon l'une quelconque des revendications 1 à 6, **caractérisé en ce qu'**il présente une excellente région spatiale pour le positionnement des mains du sujet.

8. Dispositif selon l'une quelconque des revendications 1 à 7, **caractérisé en ce que** le boîtier présente des évidements (24, 34) pour recevoir et/ou actionner un élément d'actionnement, en particulier un interrupteur de marche/arrêt (26) ou un commutateur, et/ou pour passer par une ligne d'alimentation électrique ou de données.

9. Dispositif selon l'une quelconque des revendications 1 à 8, **caractérisé en ce qu'**une paroi latérale (16, 16.1, 16.2, 18, 20, 33) ou un élément du fond de boîtier (14, 14.1) présente un dispositif de fixation pour un objet, en particulier un récipient.
